# EUROPEAN PATENT SPECIFICATION

(11) **EP 1 135 114 B1**
(45) Date of publication and mention of the grant of the patent: **07.01.2009**
(21) Application number: 99961852.3
(22) Date of filing: 29.11.1999
(51) Int. Cl.: A61K 31/02, A61P 39/06

(54) **METHOD FOR REDUCING FREE-RADICAL INDUCED INJURY IN VITRO**
METHODE ZUR IN VITRO REDUZIERUNG VON DURCH FREIE RADIKALE INDUZIERTE SCHÄDEN
PROCEDE DE REDUCTION IN VITRO DES LESIONS PROVOQUEES PAR LES RADICAUX LIBRES

(30) Priority: 01.12.1998 US 110404 P
(43) Date of publication of application: 26.09.2001
(73) Proprietor: Rotta, Alexandre T., Buffalo, NY 14209 (US); Fuhrman, Bradley P., Buffalo, NY 14222 (US); Gunnersson, Bjorn, Amherst, NY 14226 (US); Steinhorn, David M., Winnatka, IL 60093 (US); Hernan, Lynn J., Buffalo, NY 14222 (US)
(72) Inventor: Rotta, Alexandre T., Buffalo, NY 14209 (US); Fuhrman, Bradley P., Buffalo, NY 14222 (US); Gunnersson, Bjorn, Amherst, NY 14226 (US); Steinhorn, David M., Winnatka, IL 60093 (US); Hernan, Lynn J., Buffalo, NY 14222 (US)
(74) Representative: MacLean, Martin Robert
(86) International application number: PCT/US1999/028171
(87) International publication number: WO 2000/032181

(56) References cited:
- US-A- 4 523 039
- US-A- 4 993 415
- US-A- 5 292 745
- DATABASE EMBASE [Online] ELSEVIER SCIENCE PUBLISHERS, AMSTERDAM, NL; BEKYAROVA G. ET AL: "Increased antioxidant capacity, suppression of free radical damage and erythrocyte aggregability after combined application of alpha-tocopherol and FC-43 perfluorocarbon emulsion in early postburn period in rats." retrieved from STN Database accession no. 96360983 XP002179961 & ARTIFICIAL CELLS, BLOOD SUBSTITUTES, AND IMMOBILIZATION BIOTECHNOLOGY, (1996) 24/6 (629-641). ,
- DATABASE EMBASE [Online] ELSEVIER SCIENCE PUBLISHERS, AMSTERDAM, NL; OGILBY J.D.: "Cardiovascular applications of fluorocarbons: Current status and future direction a critical clinical appraisal." retrieved from STN Database accession no. 94300068 XP002179962 & ARTIFICIAL CELLS, BLOOD SUBSTITUTES, AND IMMOBILIZATION BIOTECHNOLOGY, (1994) 22/4 (1083-1096). ,
- DATABASE MEDLINE [Online] US NATIONAL LIBRARY OF MEDICINE (NLM), BETHESDA, MD, US; BEKYAROVA G ET AL: "Suppressive effect of FC-43 perfluorocarbon emulsion on enhanced oxidative haemolysis in the early postburn phase." retrieved from STN Database accession no. 97321146 XP002179963 & BURNS, (1997 MAR) 23 (2) 117-21. ,
- DATABASE EMBASE [Online] ELSEVIER SCIENCE PUBLISHERS, AMSTERDAM, NL; BANDO K. ET AL: "Oxygenated perfluorocarbon, recombinant human superoxide dismutase, and catalase ameliorate free radical induced myocardial injury during heart preservation and transplantation." retrieved from STN Database accession no. 89007860 XP002179964 & JOURNAL OF THORACIC AND CARDIOVASCULAR SURGERY, (1988) 96/6 (930-938). ,
- DATABASE MEDLINE [Online] US NATIONAL LIBRARY OF MEDICINE (NLM), BETHESDA, MD, US; ONISHCHENKO N A ET AL: "[Use of perfluorocarbon emulsion in kidney transplantation]. Ispol'zovanie perftoruglerodnoi emul'sii pri transplantatsii pochek." retrieved from STN Database accession no. 91013591 XP002181190 & KHIRURGIIA, (1990 JUN) (6) 98-103. ,
- BEKYAROVA G. ET AL: 'Suppressive effect of FC-43 perfluorocarbon emulsion on enhanced oxidative haemolysis in the early postburn phase' BURNS vol. 23, no. 2, March 1997, pages 117 - 121
- BABBITT D.G. ET AL: 'Prevention of neutrophil-mediated inhjury to endothelial cells by perfluorochemical' AMERICAN JOURNAL OF PATHOLOGY, [Online] vol. 136, 1990, pages 459 - 459 Retrieved from the Internet: <URL:http://ajp.amjpathol.org/cgi/content/a bstract/136/2/451>

## Description

### Field of the Invention

This invention relates generally to the area of in vitro free radical induced damage to cells. More particularly, this invention provides a method for in vitro reducing free radical induced damage to cells by using compositions comprising perfluorocarbons.

### Background of the Invention

### Perfluorocarbons

Perfluorocarbons (PFCs) are liquids derived from common organic compounds in which all or substantially all of the carbon-bound hydrogen atoms or other substituents have been replaced by fluorine atoms. PFCs have low surface tension and low viscosity. PFCs are clear, colorless, odorless, non-inflammable liquids that are essentially insoluble in water. PFCs are denser than water, have low surface tension and generally, low viscosity. A widely used perfluorocarbon is perfluorooctyl bromide (PFOB), also known as perflubron.

PFCs are known for their high solubility for gases. Mammals can breath certain PFCs and later resume breathing air without suffering any long-term effects. Thus, PFCs have been used for respiratory gas exchange (e.g. liquid breathing; partial liquid ventilation, Faithfull et al., U.S. Patent Nos. 5,490,498, 5,655,521; perfluorocarbon associated gas exchange, Fuhrman et al., U.S. Patent No. 5,437,272). Their high solubility of gases has made PFCs useful as blood substitutes (Riess, 1984, Artificial Organs, 8:34:56). Further, PFCs have also been used to reduce the inflammatory responses in tissues and have been recently disclosed as antiinflammatory agents (Fuhrman et al., U.S. Patent Nos. 5,470,885 and 5,733,939).

### Free Radicals

Free radicals are highly reactive structures known to cause reactions capable of damaging biomolecules in living organisms. Free radicals such as hydrogen peroxide, hydroxyl radicals, and organic free radicals are produced in vivo by enzymatic, spontaneous, radiation, and photochemical oxidation reactions, or may be generated by direct oxidative attack on cell components. Intracellular sources of free radicals include mitochondria, endoplasmic reticulum, peroxisomes, and plasma and nuclear membranes.

Free radicals can damage cell membranes by promoting peroxidation of membrane lipids, a process that has been implicated in various disease states and aging. In addition, hydroxyl radicals can cause site specific damage to DNA such as oxidation of DNA bases. Biological injury by free radicals may represent a final common pathway for inflammation in tissues. These pathways include liberation of free radicals including reactive oxygen species by cells collectively called inflammatory cells. Thus, free-radical reactions and oxidative stress appear to be basic mechanisms by which living tissues and cells are injured.

Oxidative damage to cellular mechanisms has been implicated in various conditions including photic injury to skin, carcinogenesis, aging, atherogenesis, inflammation, infection, sepsis as well as neurodegenerative, cardiovascular and respiratory diseases. Thus, there is an ongoing need to identify agents by which damage by free radicals to tissues is reduced.

Burns, Mar. 1997, 23(2), pages 117-121 describes the suppressive effect of FC-43 perfluorocarbon emulsion on enhanced oxidative haemolysis in the early post-burn phase.

EMBASE extract, AN: 96360983 (& Artificial Cell, Blood Substitutions and Immobilisation Biotechnology, (1996), 24/6, pages 629-641, describes increased antioxidant capacity, suppression of free radical damage and erthyrocyte aggrerability after combined application of alpha-tocopherol and FC-43 perfluorocarbon emulsion in the early post-burn period in rats.

EMBASE extract, AN: 94300068 (& Artificial Cell, Blood Substitutions and Immobilisation Biotechnology, (1994), 22/4, pages 1083-1096, describes cardiovascular applications of fluorocarbons.

Babbit D.C., et al., American Journal of Pathology, Vol. 136 (1990), pages 451-459, describes prevention of neutrophil-mediated injury to endothelial cells by perfluorochemical.

The present invention provides a method for protecting cells in vitro from injury, comprising contacting the cells with perfluorocarbon, wherein said perfluorocarbon protects the cells from free-radical induced injury.

### Brief Description of the Drawings

Figure 1 is a representation of free radical generation in cells as determined by fluorescence in control cells (-●-) or cells protected with perfluorocarbon (-o-) .
Figure 2 is a photomicrograph of cells wherein the fluorescence is an indicator of free radicals in cells protected with (lower panel) or without (upper panel) perfluorocarbon.
Figure 3 is a quantitative representation of the fluorescence of cells from Figure 2.

### Summary of the Invention

The present invention thus provides a method for protecting cells in vitro from injury, comprising contacting the cells with perfluorocarbon, wherein said perfluorocarbon protects the cells from free-radical induced injury. The method thus comprises the steps of exposing the cells in vitro to a pharmacologically effective amount of perfluorocarbons. Formulations comprising PFCs are useful for free-radical-effect reducing applications.

The cells may include but are not limited to epidermis, pulmonary tissue, gastrointestinal tissue and systemic tissue cells.

In another embodiment, the cells are microoganisms in culture.

### Detailed Description of the Invention

The present invention provides a method for protecting cells in in vitro from free-radical induced injury. The method involves contacting the cells with a perfluorocarbon comprising composition.

Perfluorocarbons of the present invention include straight or branched chain, or cyclic structures (Riess, supra). These molecules may have some degree of unsaturation, and may also contain bromine or hydrogenations, or they may be amine derivatives. The perfluorocarbons suitable for the present invention are similar to those useful in respiratory gas exchange methods. Suitable perfluorocarbons include FC-75, FC-77, RM-101, Hostinert 130, APF-145, APF-140, APF-125, perfluorodecalin, perfluorooctylbromide, perfluorobutyltetrahydrofuran, perfluoropropyltetrahydropyran, dimethyladamantane, trimethylbicyclononane, and combinations thereof. In a preferred embodiment, the perfluorocarbon is perflubron (PFOB)

In one embodiment of the invention, a method is provided for the use of PFCs for protecting cells in vitro from free-radical induced injury. For the method of the present invention, perfluorocarbons can be used as neat liquids, gases, or emulsions in pharmacologically effective amounts. A pharmacologically effective amount refers to an amount effective in reducing the deleterious effect of free radicals on normal cells.

PFCs can be used in formulations suitable for the specific cells in need of such treatment. For example, for using PFCs neat, the compounds can be placed into sterile, isotonic formulations and may contain additional components like conventional stabilizers and incipients. Emulsions of PFCs may be prepared by methods well known in the art. Such emulsions are typically fluorocarbon-in-water emulsions having a fluorocarbon phase and an aqueous phase. U.S. Patent No. 5,470,885 to Fuhrman et al. discloses such emulsions. For use in a gaseous form, perfluorocarbon saturated vapor or PFC droplets in the form of a mist or air-borne suspension delivered using a nebulizer or atomizer can be used.

In another embodiment, the cells to be protected from free-radical induced injury are microorganisms in culture. For this embodiment, PFCs can be used as neat liquids, as emulsions or as a component of nutrient mixtures.

Effects on free radical attack appear to require little contact with perfluorocarbon. Low concentrations and small quantities may be effective if they provide to the target of attack a quantity of perfluorocarbon that it may dissolve, store or concentrate. Thus, for certain applications, neat (100%) perfluorocarbon may be required, but for other, small quantities or low concentrations may suffice.

Cells may be contacted in vitro with PFCs prior to, simultaneously or following exposure to free radical inducing agents including, but not limited to, toxins, radiation and heat. Exposure to free-radical inducing agents may be incidental, or accidental, or predetermined.

Other object, features, and advantages of the present invention will become apparent from the following drawings and examples which are to be construed as illustrative and not restrictive.

### Example 1

### Perfluorocarbon protects Cell Monolayers against Direct in vitro oxidative injury

This embodiment of the invention demonstrates the effectiveness of PFCs against free radical injury to cells in vitro. An endothelial cell culture system was utilized to demonstrate a protective effect of PFCs. Rat pulmonary artery endothelial cells (RPAECs) were grown to confluence on optical grade culture dishes using standard technique. Cells were gown in Dulbecco's Modified Eagles Medium (high glucose) containing 1 M HEPES, 0.8 M NaOH 10% serum (calf serum). Cells were incubated at 37°C in 5% CO₂, 95% relative humidity.

An oxidative stress indicator, dichlorofluorescein diacetate (DCFDA), was used to indicate the extent of injury to cells. DCFDA is a colorless substance that permeates through the cell membrane. Fresh DCFDA (5 uM) was prepared by solubilizing 2.4 mg DCFDA in 300 ul DMSO, then diluting the solution in 1000 ml Phosphate buffered saline (PBS), pH 7.4. Immediately prior to the experiment, the culture medium was removed and the buffer containing DCFDA was added to the culture dish. Cells were incubated for 15 minutes to allow for the DCFDA probe to enter the cells. Upon excitation by contact with free radicals, the dye DCFDA becomes fluorescent and the intensity of fluorescence is directly proportional to the degree of oxidative stress suffered by the cells. After the incubation period, the DCFDA containing buffer was removed and the cells were washed with PBS (three times) to remove any residual DCFDA from the extracellular space.

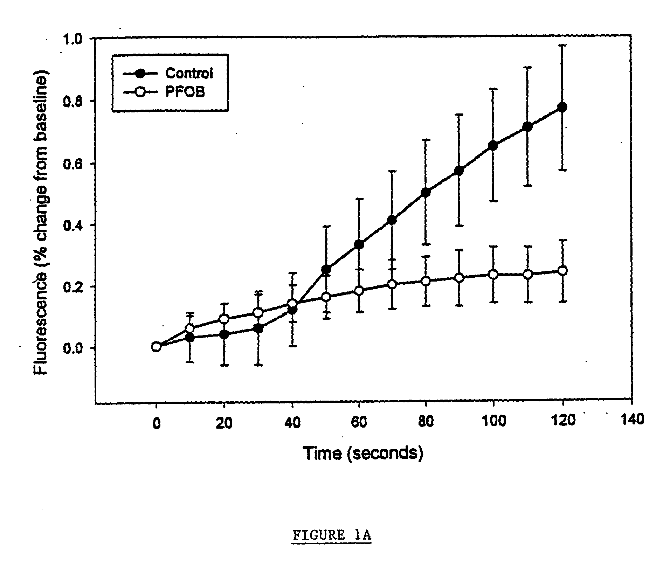

To generate free radicals, a buffer containing 10 mM H₂O₂ in PBS was used to promote oxidative stress to cell monolayers. Culture dishes were exposed to perflubron which was then either removed by evaporation or by aspiration. Thus, in one set of culture dishes (Figure 1A), cell monolayers were exposed to perflubron (PFOB) by adding 1 ml of perfluorocarbon to the culture dish. Cell monolayers not exposed to PFOB served as controls. After an incubation period of 1 minute, PFOB was aspirated from the culture dish using a fine glass pipette and cell monolayers were washed with PBS.

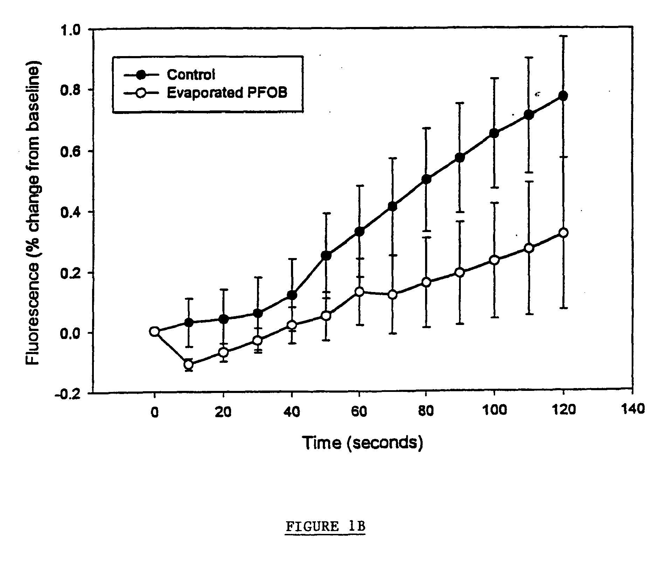

In another set of culture dishes (Figure 1B), cell monolayers were exposed to perfluorocarbon by adding 1.0 ml of perflubron to the culture dish. Cells monolayers not exposed to PFOB served as controls. After an incubation period of 1 minute, PFOB was removed from the culture dish using a glass pipette and any residual perflubron was allowed to evaporate prior to washing the cell monolayers with PBS.

Oxidative stress was initiated by the addition of the buffer containing 10 mM H₂O₂ in PBS to the cell monolayers. Oxidative stress to cells was measured by sequential imaging with a confocal laser microscope obtained at baseline and every 10 seconds for 120 seconds. The excitation filter was wet at 488 nm and the images were read at 515 nm. The images were stored as digital files and were subsequently subjected to histogram analysis by dedicated software to quantify objective differences in oxidative injury to cells per high power field.

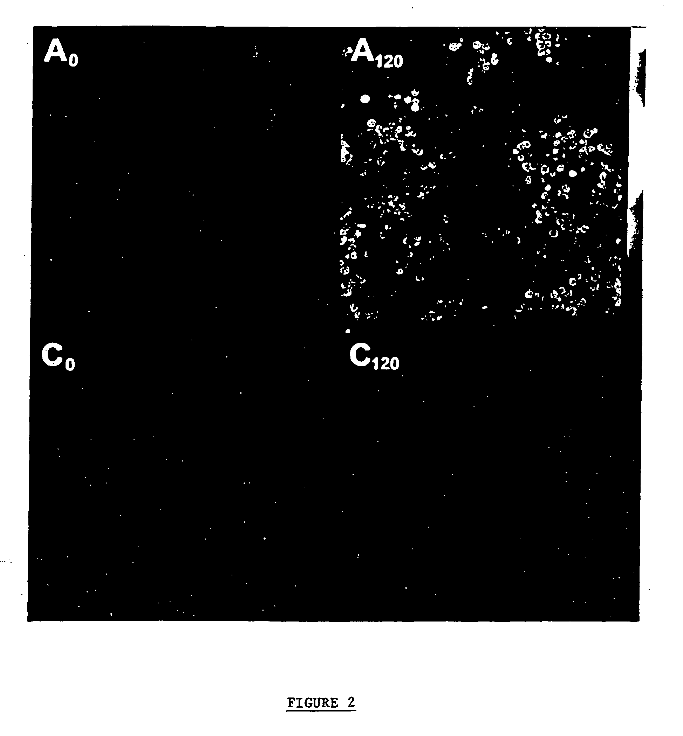

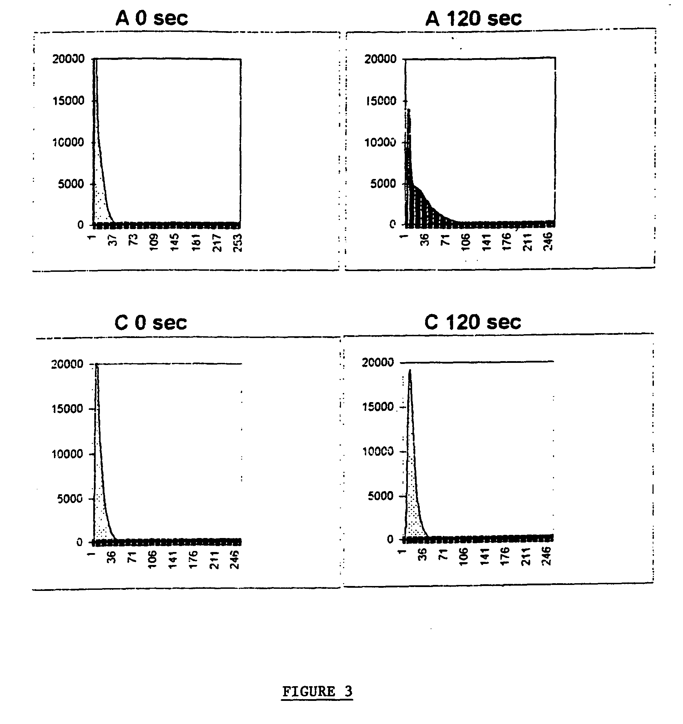

Relative fluorescence of cell monolayers exposed to perflubron (open circles) and standard buffer (closed circles) are shown in Figure 1. The upper panel (Figure 1A) depicts the experiment where perflubron was removed from the culture dishes by aspiration before application of the oxidative stress. The lower panel (Figure 1B) depicts the experiment where perflubron was removed by aspiration and allowed to evaporate. Data are expressed as mean + standard error of the mean. Digital images of representative cell monolayers from Figure 1 are shown in Figure 2. A₀ and C₀ represent the baseline fluorescence before exposure to oxidative stress. A₁₂₀ and C₁₂₀ represent fluorescence after 120 seconds of oxidative stress in cultures exposed to perfluorocarbons (C₁₂₀) or control cultures (A₁₂₀) Quantitative analysis of the fluorescence in cell cultures from Figure 2 is shown in Figure 3. A lack of fluorescence, indicating the absence free-radicals, is observed in cultures protected with PFOB (C₁₂₀) Thus, as shown in Figures 1,2 and 3, perfluorocarbon attenuates oxidative injury to cell monolayers.

## Claims

1. A method for protecting cells *in vitro* from injury, comprising contacting the cells with perfluorocarbon, wherein said perfluorocarbon protects the cells from free-radical induced injury.

2. A method according to Claim 1, wherein the cells are a cell culture.

3. A method according to Claim 2, wherein the cells are a cell monolayer.

4. A method according to Claim1, wherein the cells are microorganisms.

5. A method according to any previous claim, for protecting cells from injury caused by a stimulus selected from the group consisting of photic, radiation and chemical.

6. A method according to Claim 5, wherein the chemical stimulus is an oxidative stimulus.

7. A method according to any previous claim, wherein the perfluorocarbon is perflubron.

## Patentansprüche

1. Ein Verfahren zum Schutze von Zellen *in vitro* vor Verletzung, das das In-Kontakt-Bringen der Zellen mit Perfluorkohlenstoff umfasst, wobei der Perfluorkohlenstoff die Zellen vor Verletzung, die durch freie Radikale ausgelöst wird, schützt.

2. Ein Verfahren nach Anspruch 1, wobei die Zellen eine Zellkultur sind.

3. Ein Verfahren nach Anspruch 2, wobei die Zellen eine Zellmonoschicht sind.

4. Ein Verfahren nach Anspruch 1, wobei die Zellen Mikroorganismen sind.

5. Ein Verfahren nach einem vorherigen Anspruch zum Schutze von Zellen vor Verletzung, die vor ein Stimulans verursacht wurde, ausgewählt aus der Gruppe bestehend aus Licht, Strahlung und Chemikalien.

6. Ein Verfahren nach Anspruch 5, wobei das chemische Stimulans ein oxidatives Stimulans ist.

7. Ein Verfahren nach einem vorherigen Anspruch, wobei der Perfluorkohlenstoff Perflubron ist.

## Revendications

1. Procédé de protection de cellules *in vitro* contre des lésions, comprenant la mise en contact des cellules avec du perfluorocarbure, dans lequel ledit perfluorocarbure protège les cellules contre des lésions provoquées par des radicaux libres.

2. Procédé selon la revendication 1, dans lequel les cellules sont une culture cellulaire.

3. Procédé selon la revendication 2, dans lequel les cellules sont une monocouche cellulaire.

4. Procédé selon la revendication 1, dans lequel les cellules sont des micro-organismes.

5. Procédé selon l'une quelconque des revendications précédentes, pour protéger des cellules contre des lésions provoquées par un stimulus choisi dans le groupe constitué d'un stimulus photique, de radiation et chimique.

6. Procédé selon la revendication 5, dans lequel le stimulus chimique est un stimulus oxydatif.

7. Procédé selon l'une quelconque des revendications précédentes, dans lequel le perfluorocarbure est le perflubron.
